# EUROPEAN PATENT APPLICATION

(11) **EP 1 589 462 A2**
(43) Date of publication of application: **26.10.2005**
(21) Application number: 04018848.4
(22) Date of filing: 09.08.2004
(51) Int. Cl.: G06F 19/00

(54) **Method of managing health and system for performing the same**

(30) Priority: 21.04.2004 KR 2004027617
(71) Applicant: Chung, Chang-Jin, Uijeongbu-shi, 480-848 Gyunggi-do (KR)
(72) Inventor: Chung, Chang-Jin, Uijeongbu-shi, 480-848 Gyunggi-do (KR)
(74) Representative: Kuhnen & Wacker

(57) **Abstract**

A system of managing health comprises a medical terminal, a cellular phone, a satellite, and a medical server. The medical terminal measures health condition of a patient, and generates a measure signal having information concerning the measure result. The cellular phone generates a health information signal having information concerning health condition of the patient by using the measure signal. The satellite is connected to the cellular phone, thereby detecting in real time location of the cellular phone, and generates location information concerning the detection result. The medical server analyzes the health information signal to determine whether or not the health of the patient is normal, and manages health of the patient by using the location information and the analysis result. The system detects location of the patient by the satellite, and thus may send first-aid to the patient when the health of the patient is not normal.

## Description

### CROSS-REFERENCE TO RELATED APPLICATION

This application claims priority from Korean Patent Application No. 2004-27617, filed on April 21, 2004, the contents of which are incorporated herein by reference in its entirety.

### BACKGROUND OF THE INVENTION

### 1. Field of the Invention

The present invention relates to a method of managing health and a system for performing the same. More particularly, the present invention relates to a method of managing health and a system for performing the same capable of managing efficiently and timely a patient's health.

### 2. Description of the Related Art

A system for managing health indicates the system which manages a patient's health. When a patient visits a hospital or a doctor visits a patient at home, a conventional system for managing health diagnoses the health condition of the patient, and then provides a medical treatment corresponding to the diagnosis result to the patient. This conventional system has much room to be desired in terms of efficient medical treatment. In addition, in the conventional system, when a patient falls sick suddenly at home, the patient may not receive quick medical treatment, sometimes resulting in the patient's death though the patient might have lived if a proper emergency treatment was quickly provided. Hence, a system for managing a patient's health efficiently and timely is essential.

### SUMMARY OF THE INVENTION

It is a feature of the present invention to provide a method of managing health and a system for performing the same capable of managing a patient's health efficiently and quickly.

A system for managing health according to one embodiment of the present invention comprises a medical terminal, a cellular phone, a satellite, and a medical server. The medical terminal measures health condition of a patient, and generates a measure signal having information concerning the measure result. The cellular phone generates a health information signal having information concerning the health condition of the patient using the measure signal. The satellite is connected to the cellular phone, thereby detecting in real time the location of the cellular phone, and generates location information concerning the detection result. The medical server analyzes the health information signal to examine the patient's health, and manages the patient's health with the location information and the examination result.

A system for managing health according to one embodiment of the present invention includes a medical terminal, a satellite, and medical server. The medical terminal measures health condition of a patient, and generates a health information signal having information concerning the measure result. The satellite is connected to the medical terminal by wireless, thereby detecting in real time the location of the medical terminal, and generates location information having information concerning the detection result. The medical server analyzes the health information signal to determine the patient's health, and manages the patient's health with the analysis result and the location information.

A method of managing health according to one embodiment of the present invention includes measuring the health condition of a patient to generate a measure signal having information concerning the measure result; generating a health information signal having information concerning the health condition of the patient using the measure result; analyzing the health information signal to determine the patient's health ; detecting in real time location of the patient using a satellite, and generating location information having information concerning the detection result; and managing the patient's health in accordance with the analysis result and the location information.

As described above, the method of managing health and the system for performing the same of the present invention detects location of a patient through the satellite, and thus may provide first aid to the patient in a critical condition. By this method or system, the death rate of patients may be reduced.

In addition, the method of managing health and the system for performing the same of the present invention receive measure result concerning health condition of a patient by wireless network and provide the patient with health managing information corresponding to the measure result. Then, the patient's doctor may prescribe appropriate medical treatment and health managing method to the patient, and the patient may also manage its health efficiently.

Also, the system for managing health manages a patient's health through the cellular phone and the satellite, and so may manage the patient's health efficiently wherever the patient is located.

### BRIEF DESCRIPTION OF THE DRAWINGS

The above and other features and advantages of the present invention will become readily apparent by reference to the following detailed description in conjunction with the accompanying drawings wherein:
FIG 1 is a block diagram illustrating a system of managing health according to one embodiment of the present invention;
FIG 2 is a block diagram illustrating the medical terminal in FIG 1;
FIG 3 is a block diagram illustrating the cellular phone in FIG 1;
FIG 4 is a plan view illustrating the health information data in FIG 3;
FIG 5 is a block diagram illustrating the medical server in FIG 1;
FIG 6 is a block diagram illustrating a system for managing health according to another embodiment of the present invention;
FIG 7 is a block diagram illustrating the medical terminal in FIG 6; and
FIG 8 is a flowchart illustrating a health managing method according to one embodiment of the present invention.

### DESCRIPTION OF EMBODIMENTS

Hereinafter, preferred embodiments of the present invention will be explained in more detail with reference to the accompanying drawings.

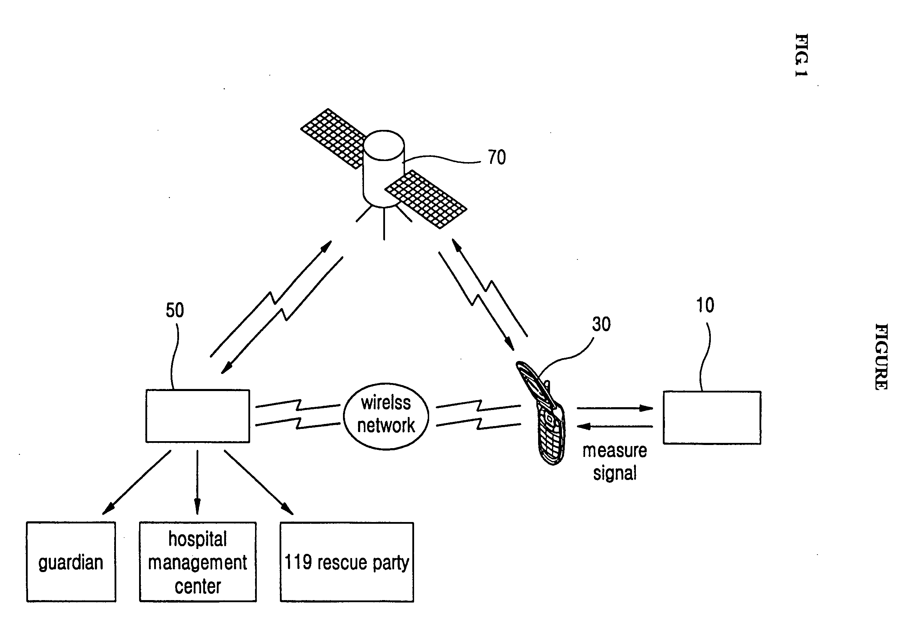

FIG 1 is a block diagram illustrating a system of managing health according to one embodiment of the present invention.

Referring to FIG 1, the system of managing health of the present invention includes a medical terminal (10), a cellular phone (30), a medical server (50) and a satellite (70).

The medical terminal (10) is adhered to the body of a patient, thereby measuring the patient's health condition (for example, electrocardiogram). In addition, the medical terminal (10) is coupled to the cellular phone (30) by wireless and transmits a measure signal having information concerning result of the measure (hereinafter, referred to as "measure result") to the cellular phone (30). Here, the medical terminal (10) is coupled to the cellular phone (30) by local area network (LAN) such as Bluetooth and others.

The cellular phone (30) is coupled to the medical terminal (10) by the LAN, and is coupled to the medical server (50) by wireless network. The cellular phone (30) transmits a health information signal including health information data to the medical server (50) using the measure signal, wherein the health information data has information concerning a patient's health condition. The cellular phone (30) according to another embodiment of the present invention may transmit the health information data to the medical server (50) through the Internet.

The medical server (50) analyzes the health information data transmitted from the cellular phone (30) to determine a patient's health condition, and then provides appropriate medical treatment corresponding to the patient's condition. Additionally, the medical server (50) detects the patient's location in real time through the satellite (70). In particular, when a measure value corresponding to the measure result is higher than predetermined reference value, the medical server (50) determines that the patient's health is abnormal. In this case, the medical server (50), which is connected to a guardian, a hospital management center, and a 119 rescue party (hereinafter, referred to as "guardian, etc."), provides them with information concerning location of the patient. When the guardian, etc. receive such information, the guardian, etc. rush to check the patient and then provide emergency services to the patient. On the other hand, when the measure value of the patient is lower than the reference value, the medical server (50) determines that the health of the patient is normal and then transmits health managing information corresponding to the health condition of the patient to the cellular phone (30) by the wireless network. Here, the health managing information may include a doctor's prescription. The medical server (50) according to another embodiment of the present invention may transmit the health managing information to the cellular phone (30) irrespective of the patient's health condition.

The cellular phone (30) displays the health managing information transmitted from the medical server (50) through a display means.

The system for managing health of the present invention detects in real time the patient's location through the satellite (70). Thus, when the medical server (50) determines that the health of a patient is not normal, the system may provide rapid and efficient emergency services to the patient. Moreover, the system detects in real time the patient's health condition by wireless network, and so may sense exactly and quickly the health condition of the patient. Hence, the system may enable the patient to receive appropriate medical treatment corresponding to the health condition of the patient.

Ms. Kyung Ae CHO states in her article, "[p]roblems in emergency treatment, and proposals to solve the problems from the perspective of urban residents" that "the death rate of first-aid patients is about 10.6%, and about 50.4% of the dead could have been prevented. Thus, it is important to organize the emergency medical system. In the Orange County, CA, U.S.A, such preventable death rate was reduced from 35% to 15% after the emergency medical system was organized. Also, in San Diego, the preventable death rate was reduced from 14% to 3% after the emergency medical system was organized."

The emergency medical system may be organized by using the system for managing the health of the present invention. The present invention's system may more efficiently aid first-aid than any other system because the system can detect in real time the location of a patient through the satellite (70). Thus, the present invention's system may reduce the death rate of emergency patients.

In case of a patient having myocardial infarction, the patient may die in 2 hours after the heart attack. In this case, the system of the present invention is connected to the guardian, etc. when the system detects that the heart rate of the patient is not normal, and so may send emergency medical service team to the patient within 2 hours from the heart attack. For another example, in case of a pregnant woman having Amniotic fluid embolism, she may die when first-aid is not provided to her within about 5 to 6 hours after attack of the Amniotic fluid embolism. In this case, the system of the present invention is connected to the guardian, etc. when the system detects that her health condition is not normal, and so may send first-aid to the patient within about 5 to 6 hours after the attack of the Amniotic fluid embolism. In other words, the system of the present invention may reduce the death rate of patients because the system enables emergency medical service team to be sent to patients in case of emergency.

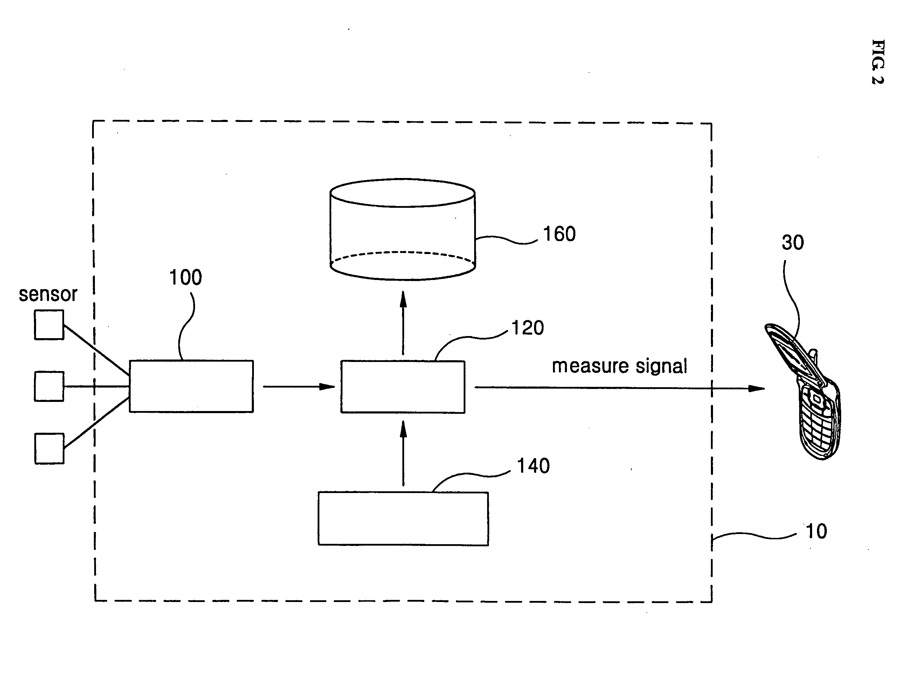

FIG 2 is a block diagram illustrating the medical terminal in FIG 1.

Referring to FIG 2, the medical terminal (10) includes a measuring section (100), a converting section (120), a medical terminal code section (140), and a medical terminal database (hereinafter, referred to as "medical terminal DB," 160).

The measuring section (100) measures the health condition of a patient, and then provides measure information concerning the measure result to the converting section (120). For example, the measuring section (100) has at least 1 sensor on the body of the patient, thereby measuring the electrocardiogram of the patient, and provides measure information concerning the measure result of the electrocardiogram to the converting section (120). The measuring section (100) may measure the health condition of a patient using other device(s) besides the sensor. However, it will be immediately obvious to those skilled in the art that many modifications for measure device used in measuring the health condition of a patient do not have any effect to the scope of the present invention.

The converting section (120) converts a medical terminal code provided from the medical terminal code section (140) and the measure information provided from the measuring section (100) into an appropriate form of measure signal having information concerning the measure result. For example, in case of measuring the electrocardiogram of a patient, the converting section (120) converts the medical terminal code and the measure information indicated as current or voltage into an appropriate form of measure signal for transmission.

The medical terminal code section (140) stores the medical terminal code by which the medical server (50) recognizes the medical terminal (10), and provides the medical terminal code to the converting section (120).

The medical terminal DB (160) receives the measure information from the converting section (120), and stores the measure information. The medical terminal DB (160) according to one embodiment of the present invention may store the health managing information transmitted from the medical server (50) via the cellular phone (30).

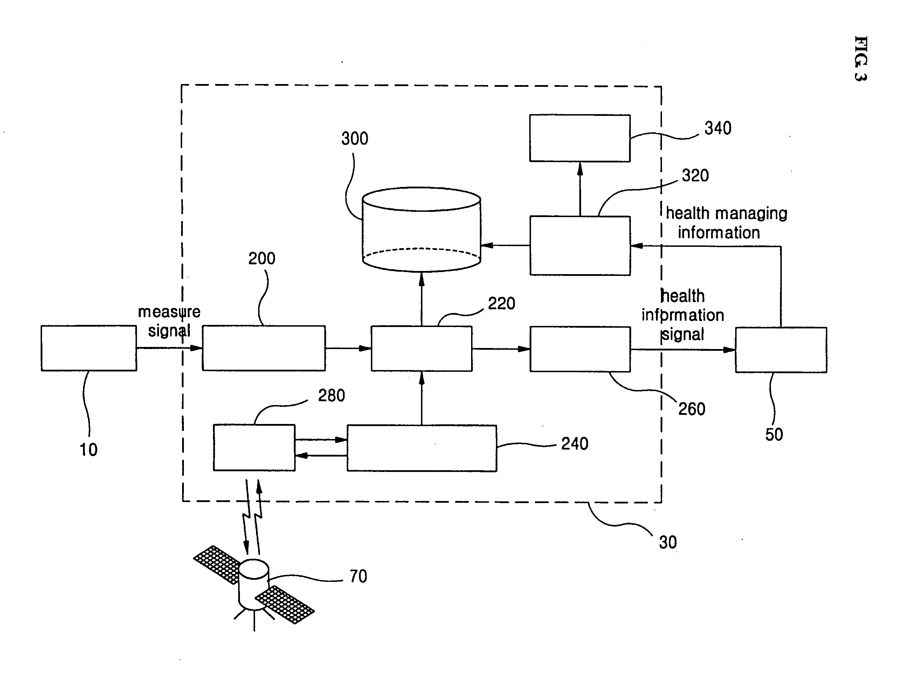

FIG. 3 is a block diagram illustrating the cellular phone in FIG 1.

Referring to FIG 3, the cellular phone (30) includes a measure signal receiver (200), an information generating section (220), a cellular phone code section (240), an information transmitter (260), a satellite section (280), a cellular phone database (hereinafter, referred to as "cellular phone DB," 300), a health managing information receiver (320), and a displaying section (340).

The measure signal receiver (200) receives the measure signal from the medical terminal (10), and provides the measure signal to the information generating section (220).

The cellular phone code section (240) includes various kinds of code, and provides the code to the information generating section (220). For example, the cellular phone code section (240) includes a first code to be recognized by the medical server (50) and a second code to be recognized by the satellite (70).

The information generating section (220) combines the measure result of the health condition of a patient included in the measure signal with the first code, thereby generating health information data. In addition, the information generating section (220) transmits the health information data to the information transmitter (260) and the cellular phone DB (300).

The information transmitter (260) transmits the health information signal including the health information data to the medical server (50).

The satellite section (280) receives the second code from the cellular phone code section (240). The satellite (70) is connected to the satellite section (280) by wireless, thereby recognizing the second code through the satellite section (280). As a result, the satellite (70) detects the location of the cellular phone (30), that is, the location of the patient. The satellite (70) according to another embodiment of the present invention detects the location of the medical terminal (10) through the cellular phone (30) connected to the medical terminal (10) by the LAN. As a result, the satellite (70) may detect in real time the location of the medical terminal (10), that is, the location of the patient.

The health managing information receiver (320) receives the health managing information from the medical server (50), and provides the health managing information to the displaying section (340) and the cellular terminal DB (300).

The cellular terminal DB (300) stores the health information data and the health managing information.

The displaying section (340) displays the health managing information to the patient. As a result, the patient recognizes his health condition, and may take active and efficient care of his health in accordance with prescription of a doctor displayed on the displaying section (340).

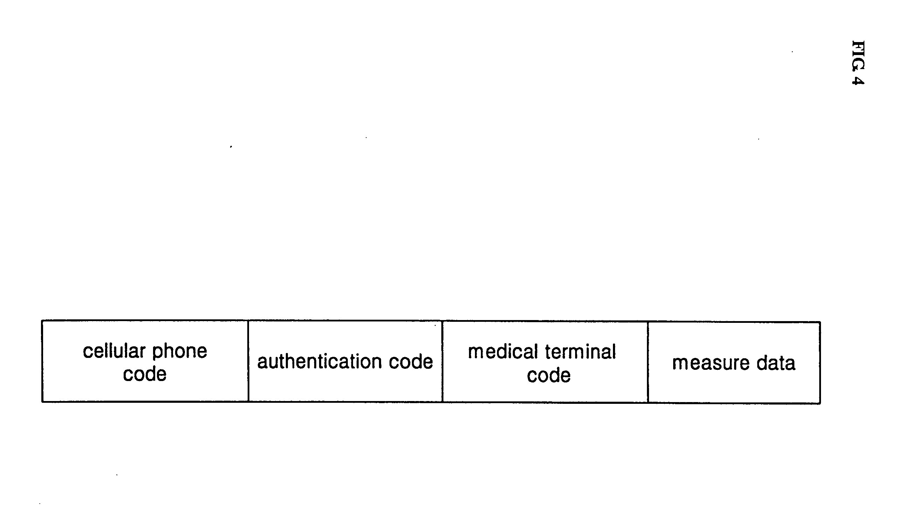

FIG 4 is a plane view illustrating the health information data in FIG 3.

Referring to FIG 4, the health information data includes a cellular phone code, an authentication code, a medical terminal code, and measure data. Here, the cellular phone code indicates the code through which the medical server (50) recognizes the cellular phone (30), and the authentication code means the code through which the medical server (50) recognizes the patient. In addition, the medical terminal code represents the code through which the medical server (50) recognizes the medical terminal (10), and the measure data includes the information concerning the measure result.

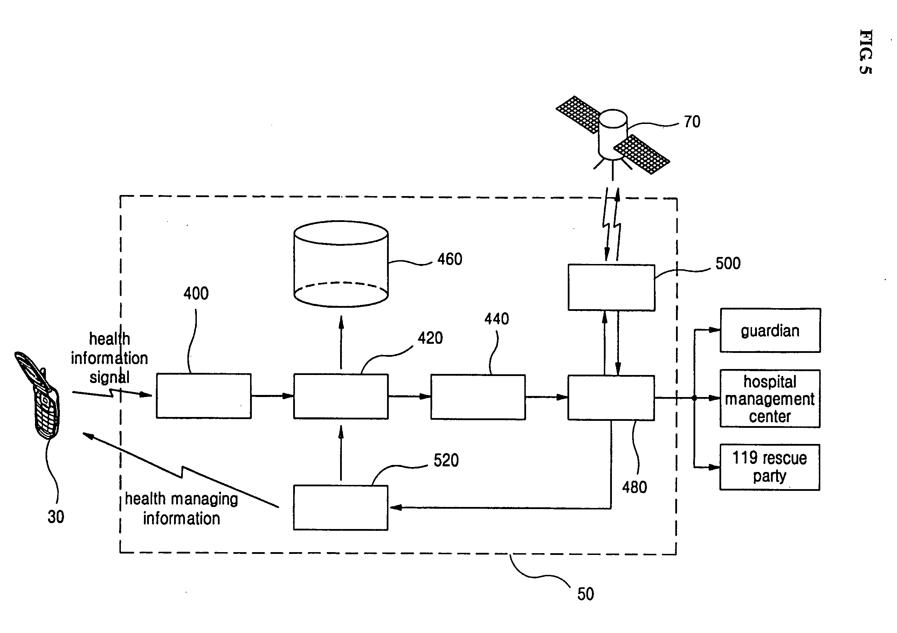

FIG 5 is a block diagram illustrating the medical server in FIG 1.

Referring to FIG 5, the medical server (50) includes a health information receiver (400), an information-analyzing section (420), a condition-examining section (440), a server database (hereinafter, referred to as "server DB", 460), a controlling section (480), a location-detecting section (500), and a health managing-information providing section (520).

The health information receiver (400) receives health information signal transmitted from the cellular phone (30) through an antenna, etc., and transmits the received health information signal to the information analyzing section (420).

The information analyzing section (420) analyzes information concerning the measure result included in the health information signal, and provides the result of analysis (hereinafter, referred to as "analysis result") to the server DB (460) and the condition-examining section (440). However, it will be immediately obvious to those skilled in the art that many modifications of the above analyzing method are possible.

The condition-examining section (440) compares measure value corresponding to the analysis result with a predetermined reference value to determine whether or not the health of the patient is normal. In particular, when the measure value is higher than the reference value, the condition-examining section (440) determines that the health of the patient is not normal. Whereas, when the measure value is lower than the reference value, the condition-examining section (440) determines that the health of the patient is normal.

The location-detecting section (500) is connected to the satellite (70) by wireless, and receives in real time information concerning the location of the patient from the satellite (70). Additionally, the location-detecting section (500) transmits the information concerning the location of the patient to the controlling section (480).

When the condition-examining section (440) determines that the health of the patient is not normal, the controlling section (480) transmits information concerning the location of the patient and information concerning the health condition of the patient to the guardian, etc. As a result, the guardian, etc. may quickly move to provide emergency services to the patient. In addition, the controlling section (480) transmits the information concerning health condition of the patient to the health managing information providing section (520).

The health managing information providing section (520) provides the health managing information corresponding to health condition of the patient to the cellular phone (30). The health managing information according to one embodiment of the present invention includes prescription of a doctor.

The server DB (460) stores analysis result concerning health condition of the patient. The server DB (460) according to one embodiment of the present invention may store health information for many patients by using various storage methods together.

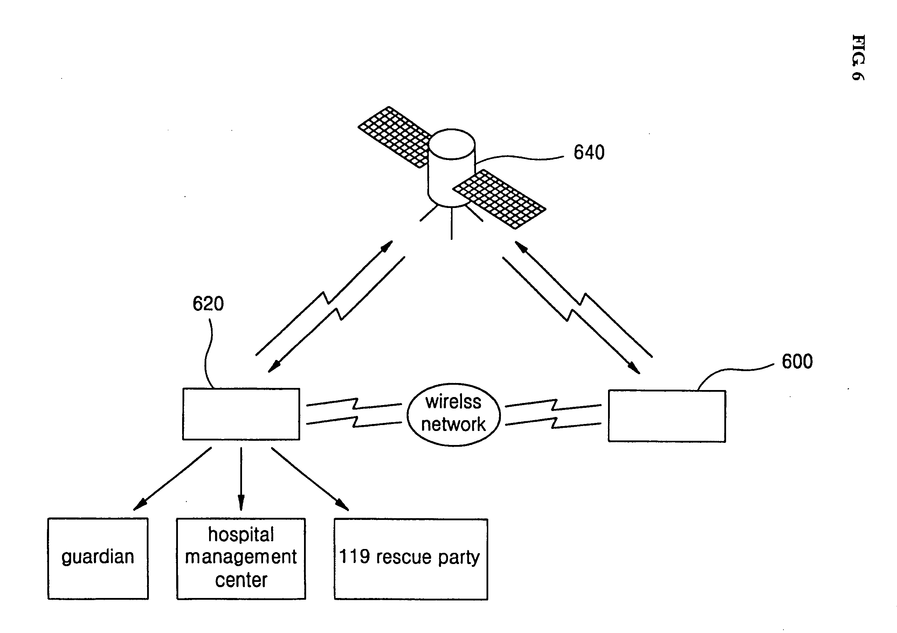

FIG 6 is a block diagram illustrating a system for managing health according to another embodiment of the present invention.

Referring to FIG 6, the system of the present invention includes a medical terminal (600), a medical server (620), and a satellite (640).

The medical terminal (600) is adhered to the body of a patient, thereby measuring health condition of the patient, and provides the measure result to the medical server (620) by wireless network.

The satellite (640) is connected to the medical terminal (600) by wireless, and detects in real time the location of the medical terminal (600), i.e. the location of the patient. Moreover, the satellite (640) transmits the location of the medical terminal (600) to the medical server (620).

The medical server (620) analyzes the measure result provided from the medical terminal (600) to determine whether or not the health of the patient is normal. In addition, when the medical server (620) determines that the health of the patient is not normal, the medical server (620) transmits information concerning location of the patient and information concerning the health condition of the patient to the guardian, etc. As a result, the guardian, etc. may move rapidly to provide emergency services to the patient. The medical server (620) transmits health managing information corresponding to health condition of the patient to the medical terminal (600) by wireless network.

The medical terminal (600) according to one embodiment of the present invention receives and displays the health managing information.

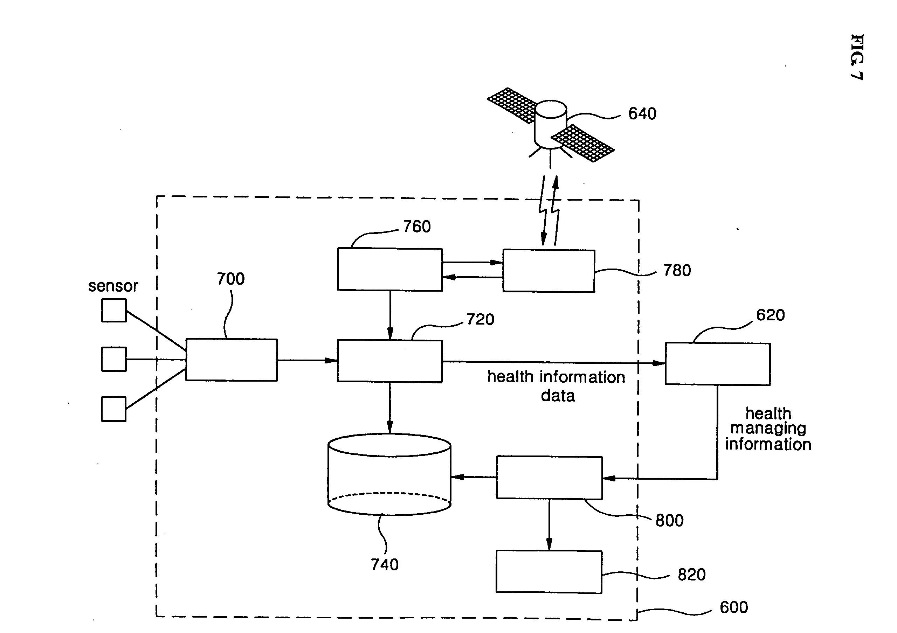

FIG 7 is a block diagram illustrating the medical terminal in FIG 6.

Referring to FIG 7, the medical terminal (600) includes a measuring section (700), an information generating section (720), a medical terminal database (hereinafter, referred to as "medical terminal DB," 740), a code section (760), a satellite section (780), a health managing information receiver (800), and a displaying section (820).

The measuring section (700) is adhered to the body of a patient, thereby measuring health condition of the patient, and transmits the measure result to the information generating section (720).

The code section (760) stores various kinds of codes, and provides the codes to the information generating section (720) and the satellite section (780).

The information generating section (720) combines the measure result and the code to generate health information data concerning the measure result. In addition, the information generating section (720) transmits the health information data to the medical server (620) and the medical terminal DB (740).

The health managing information receiver (800) receives the health managing information transmitted from the medical server (620), and transmits the received health managing information to the medical terminal DB (740) and the displaying section (820).

The displaying section (820) displays the health managing information to the patient.

The medical terminal DB (740) stores the health information data and the health managing information.

The satellite section (780) receives a medical terminal code to be recognized by the satellite (640) from the code section (760) and stores the medical terminal code. As a result, the satellite (640) recognizes the medical terminal code to detect the location of the medical terminal (600), i.e. the location of the patient.

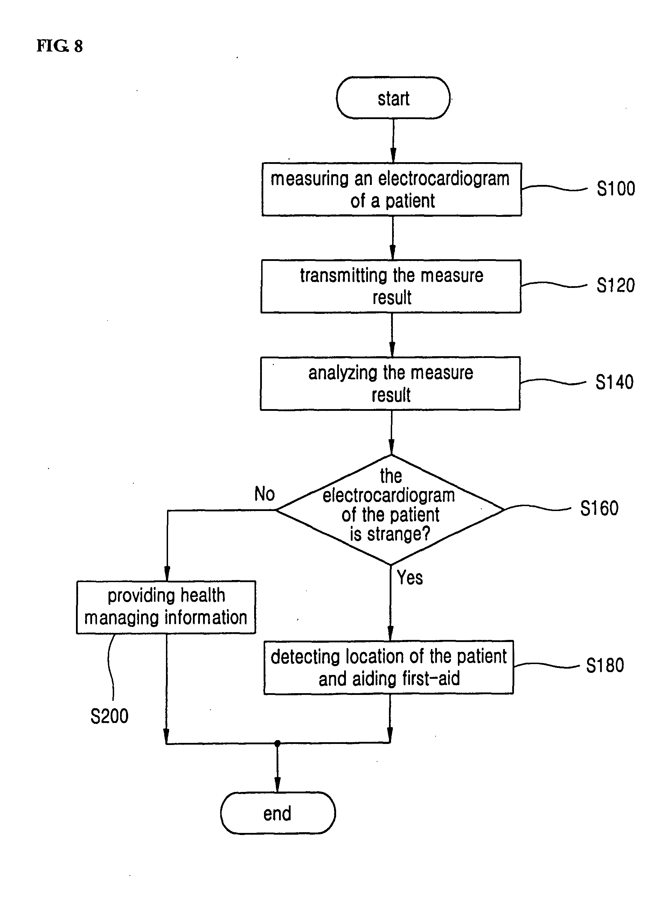

FIG 8 is a flowchart illustrating a health managing method according to one embodiment of the present invention. Hereinafter, for example, the measuring section (100 and 700) measures the electrocardiogram of the patient.

Referring to FIG 8, in step S100, the measuring section (100 and 700) measures the electrocardiogram of the patient through a plurality of sensors.

In step S120, the measure result is transmitted to the medical server (50 and 620).

In step S140, the medical server (50 and 620) analyzes the measure result.

In step S160, the medical server (50 and 620) compares measure value corresponding to the measure result with reference value to determine whether or not the health of the patient is normal.

In step S180, when the medical server (50 and 620) determines that the health of the patient is not normal, the medical server (50 and 620) transmits the information concerning the location of the patient and the information concerning the health condition of the patient to the guardian, etc. As a result, the guardian, etc. move rapidly to provide emergency services to the patient.

In step S200, when the medical server (50 and 620) determines that the health of the patient is normal, the medical server (50 and 620) provides the health managing information.

From the above preferred embodiments for the present invention, it is noted that modifications and variations thereto can be made by a person skilled in the art in light of the above teachings. Therefore, it should be understood that changes may be made for a particular embodiment of the present invention within the scope and spirit of the present invention outlined by the appended claims.

## Claims

1. A system for managing health comprising:
a medical terminal for measuring the health condition of a patient, and generating a measure signal having an information concerning the measure result;
a cellular phone for generating a health information signal having an information concerning the health condition of the patient using the measure signal;
a satellite connected to the cellular phone, thereby detecting in real time the location of the cellular phone, and generating a location information concerning the detection result; and
a medical server for analyzing the health information signal to determine whether or not the health of the patient is normal and managing the health of the patient by using the location information and the analysis result.

2. The system of claim 1, wherein the medical terminal measures the electrocardiogram of the patient.

3. The system of claim 1, wherein the medical server transmits health managing information corresponding to health condition of the patient to the cellular phone in accordance with the analysis result.

4. The system of claim 3, wherein the cellular phone includes a displaying section for displaying the health managing information.

5. The system of claim 1, wherein the medical server provides the location information and an information concerning the analysis result to a guardian, a hospital management center, or a 119 rescue party when the medical server determines that the health of the patient is not normal.

6. The system of claim 1, wherein the cellular phone is connected to the medical server by wireless network, and is connected to the medical terminal by local area network.

7. A system for managing health comprising:
a medical terminal for measuring the health condition of a patient, and generating a health information signal having an information concerning the measure result;
a satellite connected to the medical terminal by wireless, thereby detecting in real time the location of the medical terminal and generating location information having information concerning the detection result; and
a medical server for analyzing the health information signal to determine whether or not the health of the patient is normal, and managing the health of the patient by using the analysis result and the location information.

8. The system of claim 7, wherein the medical server transmits health managing information corresponding to the analysis result to the medical terminal.

9. The system of claim 8, wherein the medical terminal includes:
an information generating section for measuring health condition of the patient to generate the health information signal;
a displaying section for displaying the health managing information thereon;
a medical terminal database for storing the information concerning the measure result and the health managing information; and
a satellite section connected to the satellite by wireless, thereby providing the location information to the medical server.

10. A method of managing health comprising:
measuring the health condition of a patient to generate a measure signal having information concerning the measure result;
generating a health information signal having an information concerning health condition of the patient by using the measure result;
analyzing the health information signal to determine whether or not the health of the patient is normal;
detecting in real time location of the patient by using a satellite, and generating location information having an information concerning the detection result; and
managing health of the patient in accordance with the analysis result and the location information.

11. The method of claim 10, wherein the health of the patient is managed by:
providing health managing information to the patient in accordance with the analysis result; and
providing the location information and information concerning the analysis result to a guardian, a hospital management center or a 119 rescue party in case it is determines that the health of the patient is not normal, thereby providing emergency services to the patient.

12. The method of claim 10, wherein an electrocardiogram of the patient is measured.
